# EUROPÄISCHE PATENTSCHRIFT

(11) **EP 2 133 047 B1**
(45) Veröffentlichungstag und Bekanntmachung des Hinweises auf die Patenterteilung: **16.02.2011**
(21) Anmeldenummer: 09161494.1
(22) Anmeldetag: 29.05.2009
(51) Int. Cl.: A61F 5/44

(54) **Harnauffangbeutel**
Urine receptacle
Sac récupérateur d'urine

(30) Priorität: 13.06.2008 DE 202008007958 U
(43) Veröffentlichungstag der Anmeldung: 16.12.2009
(73) Patentinhaber: Jaeniche, Wilhelm, 77694 Kehl-Leutesheim (DE)
(72) Erfinder: Grundke, Reinhold, 84489 Burghausen (DE)
(74) Vertreter: Hofstetter, Alfons J.

(56) Entgegenhaltungen:
- DE-U1-202004 010 369
- GB-A- 2 255 548

## Beschreibung

Die vorliegende Erfindung betrifft einen Harnauffangbeutel mit mindestens einem Beutelraum zur Aufnahme des Harns und mindestens einem Einlassanschluss der in den Beutelraum mündet.

Harnauffangbeutel der eingangs genannten Art sind aus dem Stand der Technik bekannt. So werden zum Beispiel so genannte Bettbeutel an verschiedenartige Harnauffangsysteme angeschlossen, wobei die Bettbeutel lösbar an einem Patientenbett befestigt sind. Auch tragbare Harnauffangbeutel, so genannte Beinbeutel sind bekannt. Derartige Beinbeutel werden gewöhnlich als Urinbeutel bei Personen eingesetzt, bei denen der Urin per Urinalkondom oder Katheter abgeführt werden soll. Bekannte Harnauffangbeutel werden als Einmalprodukt zur einmaligen Verwendung oder als wiederverwendbare Ausführung mit einem Auslassventil oder ähnlichem angeboten. Beutel zur Einmal-Benutzung verursachen relativ hohe Kosten und ein Entsorgungsproblem. Zudem besteht die Gefahr einer erhöhten Keimbildung durch eine zu lange Benutzung der Beutel. Auch die bekannten mehrfach verwendbaren Harnauffangbeutel bieten hierfür keine Lösung an, da die geforderte Keimfreiheit vor jeder Benutzung durch eine zum Beispiel maschinelle Reinigung nicht gewährleistet werden kann. Dies liegt insbesondere darin begründet, dass bekannte Harnauffangbeutel Nähte aufweisen, an und in denen sich Bakterien ohne weiteres festsetzen können, die nur sehr schwer oder überhaupt nicht entfernt bzw. abgetötet werden können. Dadurch ist ein hohes Infektionsrisiko für den Patienten, insbesondere bei der Mehrfachverwendung der bekannten Harnauffangbeutel gegeben. Die DE 20 2004 010369 U offenbart einen Urinauffangbehälter bestehend aus einem stabilen Behälterkorpus und einem lösbaren, den Behälterkorpus schließenden Deckel, wobei der Behälterkorpus eine kantenfreie Form aufweist. Durch die kantenfreie Ausbildung des Behälterkorpus soll eine vollständige Reinigung möglich sein, so dass keine Infektionskeime im Behälter verbleiben. Nachteilig an diesem bekannten Urinauffangbehälter ist jedoch, dass der Behälterkorpus starr ausgebildet ist. Dadurch kann dieser Behälter nur als Bettbeutel verwendet werden. Eine Verwendung als Beinbeutel ist nicht möglich. Zudem ist er aufgrund der vorhandenen Deckel-/Korpus-Konstruktion konstruktiv aufwändig herzustellen, wodurch relativ hohe Herstellungskosten entstehen.

Es ist daher Aufgabe der vorliegenden Erfindung, einen Harnauffangbeutel der eingangs genannten Art bereitzustellen, der relativ kostengünstig in der Herstellung ist, als Bett- und Beinbeutel und zudem mehrfach verwendbar ist.

Zur Lösung dieser Aufgaben dienen ein gattungsgemäßer Harnauffangbeutel gemäß den Merkmalen des Anspruchs 1.

Vorteilhafte Ausgestaltungen sind in den jeweiligen Unteransprüchen beschrieben.

Ein erfindungsgemäßer Harnauffangbeutel weist mindestens einen Beutelraum zur Aufnahme des Harns und mindestens einen Einlassanschluss auf, der in dem Beutelraum mündet. Zudem ist der Beutelraum nahtfrei und umstülpbar ausgebildet und besteht aus einem flexiblen, flüssigkeitsundurchlässigen und folienartigen Kunststoffmaterial. Die nahtfreie Ausgestaltung des Beutelraums sowie die Möglichkeit den Harnauffangbeutel bzw. den Beutelraum umzustülpen ermöglichen es, dass einerseits die Gefahr einer Festsetzung von Bakterien innerhalb des Beutelraums an üblicherweise vorhandenen Nähten zuverlässig verhindert wird. Zudem ergibt sich durch die Umstülpbarkeit des Beutelraums bzw. des Harnauffangbeutels die Möglichkeit einer optimalen, insbesondere maschinellen Reinigung des gebrauchten Hamauffangbeutels. Somit kann eine Keimfreiheit des gereinigten erfindungsgemäßen Harnauffangbeutels erreicht werden. Zudem ergibt sich durch die Verwendung eines flexiblen und folienartigen Kunststoffmaterials vorteilhafterweise die Möglichkeit, dass der erfindungsgemäße Harnauffangbeutel sowohl als Bett- als auch als Beinbeutel verwendet werden kann. Zudem ist die Herstellung des Harnauffangbeutels aus diesen Materialen relativ kostengünstig zu realisieren. Dabei kann das Kunststoffmaterial zum Beispiel eine Schlauchfolie sein, wobei die Schlauchfolie zum flüssigkeitsdichten Abschluss und zur Ausbildung des Beutelraums an einem bodenseitigen Ende des Beutelraums nahtfrei verschweißt ist.

In einer weiteren vorteilhaften Ausgestaltung des erfindungsgemäßen Harnauffangbeutels ist der Beutelraum mittels mindestens einer Verschlussvorrichtung verschließbar ausgebildet. Dabei kann die Verschlussvorrichtung aus mindestens einer in einer ersten Beutelwand ausgebildeten linienförmigen und hervorstehenden Prägung besteht, wobei die Prägung in eine entsprechende Nut in einer der ersten Beutelwand gegenüberliegenden zweiten Beutelwand lösbar eindrückbar ist. Durch die Ausbildung einer Verschlussvorrichtung wird einerseits gewährleistet, dass kein Harn aus dem Harnauffangbeutel austreten kann. Andererseits kann der Harnauffangbeutel geöffnet werden, so dass dieser einerseits schnell entleert werden kann und andererseits eine einfache Reinigung des Beutelraums möglich wird.

In einer weiteren vorteilhaften Ausgestaltung des erfindungsgemäßen Harnauffangbeutels weist dieser mindestens einen Auslassanschluss zum Anschluss eines Schlauchstücks zur Ableitung des Harns aus dem Beutelraum auf. Dabei kann der Auslassanschluss oder das an dem Auslassanschluss angeordnete Schlauchstück mittels eines abnehmbaren oder nicht-abnehmbaren Clipverschlusses verschiebbar sein. Zudem besteht die Möglichkeit, dass das Schlauchstück einstückig mit dem Auslassanschluss verbunden ist. Durch die Ausbildung eines Auslassanschlusses ergibt sich eine vorteilhafte und einfache Möglichkeit den Harn aus dem Beutelraum abzulassen. Wird ein abnehmbarer Clipverschluss verwendet, so kann dieser vorteilhafterweise mehrfach, auch bei anderen Harnauffangbeuteln verwendet werden.

In einer weiteren vorteilhaften Ausgestaltung des erfindungsgemäßen Harnauffangbeutels weist der Einlassanschluss ein Ventil zur Verhinderung eines Harnrückflusses aus dem Beutelraum auf. Durch diese erfindungsgemäße Maßnahme ist gewährleistet, dass der Rückfluss von Harn aus dem Beutelraum in Richtung des Urogenitaltrakts sicher verhindert wird. Dadurch wird das Infektionsrisiko erheblich minimiert, ein Aufstieg von Bakterien aus dem Harnauffangbeutel in den Urogenitaltrakt über eine katheterartige Verbindung zwischen dem Einlassanschluss und dem Urogenitaltrakt wird nahezu ausgeschlossen.

In einer weiteren vorteilhaften Ausgestaltung der Erfindung weist der Beutelraum bodenseitig gerundete Eckbereiche auf. Durch die Ausbildung dieser gerundeten Eckbereiche ergibt sich zuverlässig eine nahezu vollständige Leerung des Harnauffangbeutels.

In einer weiteren vorteilhaften Ausgestaltung der Erfindung weist der Harnauffangbeutel in einem dem Beutelraum nach oben abschließenden Bereich mindestens eine Öffnung zur Aufnahme einer hakenförmigen Halterung auf. Die Halterung ist dabei entweder an einem Patientenbett angeordnet oder an einer entsprechenden Beinhalterung für den Hamauffangbeutel. Auch andere Befestigungsvorrichtungen zur lösbaren Befestigung des Harnauffangbeutels an einem Patientenbett oder ähnlichem sind denkbar. Es kann auch eine entsprechende Beutelhalterung in einer Innentasche einer Hose im Oberschenkelbereich eines Patienten ausgebildet sein.

In einer weiteren vorteilhaften Ausgestaltung der Erfindung weist der Harnauffangbeutel in einem dem Beutelraum nach oben abschließenden Bereich mindestens eine überstehende Bedienlasche auf. Durch die Bedienlasche wird vorteilhafterweise einerseits die Öffnung des Beutelraums vereinfacht, einerseits lässt sich der Harnauffangbeutel leichter handhaben.

Weitere Einzelheiten, Merkmale und Vorteile der Erfindung ergeben sich aus zwei in den folgenden Zeichnungen dargestellten Ausführungsbeispielen. Es zeigen:
- Figur 1: eine schematische Darstellung eines erfindungsgemäßen Harnauffang- beutels gemäß einer ersten Ausführungsform;
- Figur 2: eine schematische Darstellung eines erfindungsgemäßen Harnauffang- beutels gemäß einer zweiten Ausführungsform; und
- Figur 3: eine schematische Darstellung eines Clipverschlusses des erfindungs- gemäßen Harnauffangbeutels gemäß Figur 2.

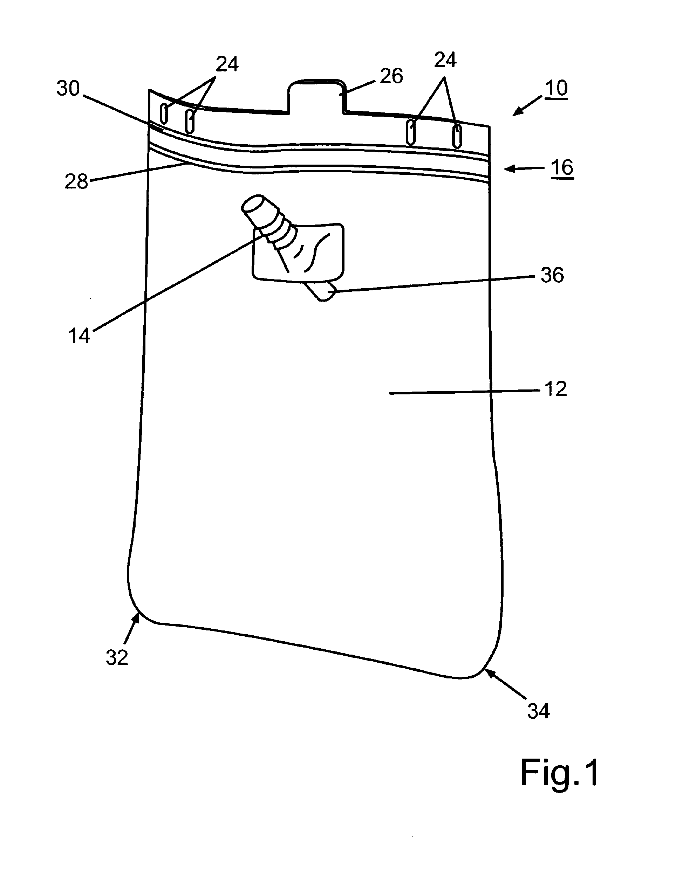

Figur 1 zeigt eine schematische Darstellung eines Harnauffangbeutels 10 gemäß einer ersten Ausführungsform. Der Harnauffangbeutel 10 besteht üblicherweise aus Kunststoff oder einem anderen geeigneten Plastikmaterial. Weitere Materialien sind aber ebenfalls denkbar. Der Harnauffangbeutel 10 weist zudem einen Beutelraum 12 auf, der durch eine umlaufende Beutelwand flüssigkeitsdicht begrenzt wird. Des Weiteren ist ein Einlassanschluss 14, der in den Beutelraum 12 mündet, ausgebildet. In dem Beutelraum 12 kann auch ein so genannter Superabsorber angeordnet sein (nicht dargestellt).

Der Harnauffangbeutel 10 bzw. der Beutelraum 12 ist nahtfrei und umstülpbar ausgebildet. In dem dargestellten Ausführungsbeispiel ist das genannte flexible und flüssigkeitsundurchlässige Kunststoffmaterial eine Schlauchfolie, wobei die Schlauchfolie zum flüssigkeitsdichten Abschluss und zur Ausbildung des Beutelraums 12 an einem bodenseitigen Ende des Beutelraums 12 nahtfrei verschweißt ist. Zudem weist der Beutelraum 12 bodenseitig gerundete Eckbereiche 32, 34 auf. Die nahtfreie und umstülpbare Ausbildung des Harnauffangbeutels 10 bzw. des Beutelraums 12 gewährleistet, dass eine vollständige Reinigung des Harnauffangbeutels 10 möglich ist. Zudem wird zuverlässig das Festsetzen von Bakterien im Beutelraum 12 verhindert.

Des Weiteren erkennt man, dass der Beutelraum 12 mittels einer Verschlussvorrichtung 16 verschließbar ausgebildet ist. Die Verschlussvorrichtung 16 besteht dabei aus zwei parallel zueinander im oberen, dem bodenseitigen Ende des Beutelraums gegenüberliegenden Bereich in einer ersten Beutelwand ausgebildeten linienförmigen und hervorstehenden Prägungen 28, 30. Die Prägungen 28, 30 sind in entsprechende Nute in einer der ersten Beutelwand gegenüberliegenden zweiten Beutelwand lösbar eindrückbar. Die Prägungen 28, 30 verlaufen dabei über die gesamte Breite des Harnauffangbeutels 10 bzw. des Beutelraums 12. Der Einlassanschluss 14 ist bis in den Beutelraum über eine Anschlussverlängerung 36 hin verlängert. Dadurch ergibt sich eine gleichmäßige Befüllung des Beutelraums 12 mit Harn. Der Einlassanschluss 14 ist zudem derart ausgebildet, dass daran eine schlauchartige Verbindung, zum Beispiel ein Katheter aufsteckbar und befestigbar ist. Zudem kann der Einlassanschluss 14 ein Ventil zur Verhinderung eines Harnrückflusses aus dem Beutelraum 12 aufweisen. Des Weiteren erkennt man, dass der Harnauffangbeutel 10 in den dem Beutelraum 12 nach oben abschließenden Bereichen mehrere Öffnungen 24 zur Aufnahme einer hakenförmigen Halterung, zum Beispiel einer an einem Patientenbett angeordneten Halterung aufweist. Zudem weist der Harnauffangbeutel 10 in diesem Bereich eine überstehende Bedienlasche 26 auf.

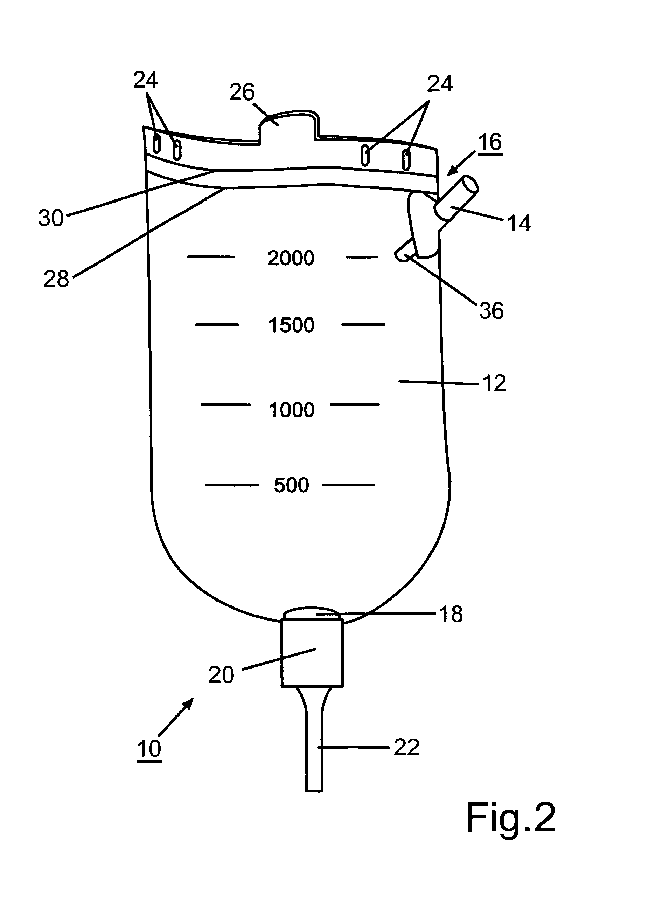

Figur 2 zeigt eine schematische Darstellung eines Harnauffangbeutels 10 gemäß einer zweiten Ausführungsform. Im Unterschied zu der in Figur 1 gezeigten ersten Ausführungsform weist dieser Harnauffangbeutel 10 einen Auslassanschluss 18 zum Anschluss eines Schlauchstücks 22 zur Ableitung des Harns an dem Beutelrand 12 auf. Das entsprechende Ende des Schlauchstücks 22 kann dabei entweder einstückig mit dem Auslassanschluss 18 verbunden sein oder auf diesen aufsteckbar und befestigbar ausgebildet sein. Des Weiteren erkennt man, dass das an dem Ausschlussanlass 18 angeordnete Schlauchstück 22 mittels eines abnehmbaren Clipverschlusses 20 verschließbar ist. Des Weiteren ist der Harnauffangbeutel 10 gemäß dem in Figur 2 gezeigten Ausführungsbeispiel an seinem bodenseitigen Ende halbkreisförmig ausgebildet. Die übrigen Merkmale entsprechen denjenigen des in Figur 1 beschriebenen Ausführungsbeispiels.

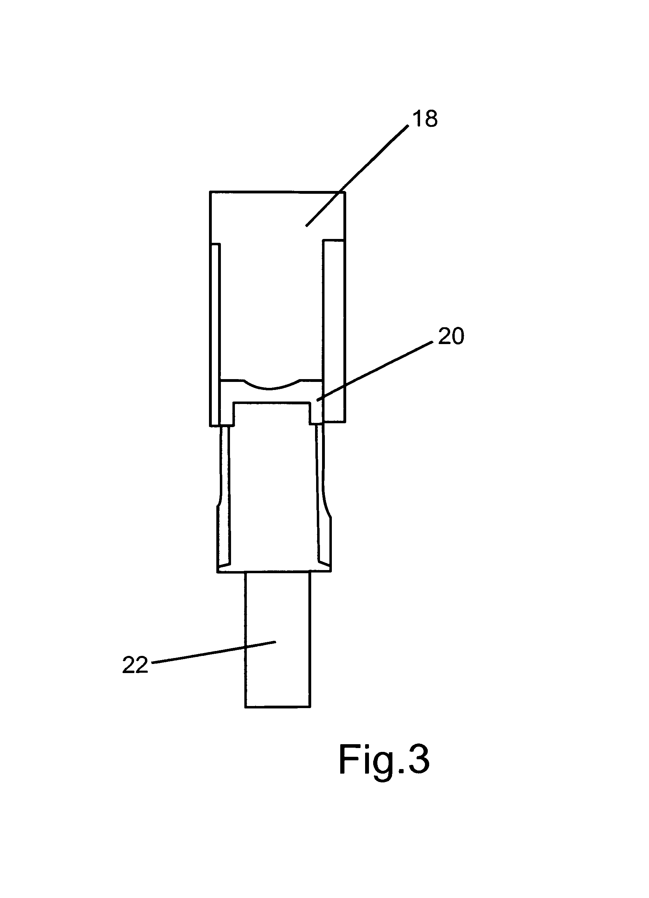

Figur 3 zeigt eine schematische Darstellung des Clipverschlusses 20 des Harnauffangbeutels 10 gemäß Figur 2. Man erkennt die clipartige Ausgestaltung des Clipverschlusses 20, wobei in dem dargestellten Ausführungsbeispiel der Clipverschluss 20 auf den Auslassanschluss 16 aufsteckbar ist und im Bereich des Schlauchstückes 22 den Harnabfluss sperrt oder öffnet.

## Patentansprüche

1. Harnauffangbeutel mit mindestens einem Beutelraum (12) zur Aufnahme des Harns und mindestens einem Einlassanschluss (14) der in den Beutelraum (12)mündet, wobei der Beutelraum nahtfrei ausgebildet ist,
**dadurch gekennzeichnet,**
**dass** der Beutelraum (12) umstülpbar ausgebildet ist und aus einem flexiblen, flüssigkeitsundurchlässigen und folienartigen Kunststoffmaterial besteht.

2. Harnauffangbeutel nach Anspruch 1,
**dadurch gekennzeichnet,**
**dass** das Kunststoffmaterial eine Schlauchfolie ist, wobei die Schlauchfolie zum flüssigkeitsdichten Abschluss und zur Ausbildung des Beutelraums (12) an einem bodenseitigen Ende des Beutelraums (12) nahtfrei verschweißt ist.

3. Harnauffangbeutel nach einem der vorhergehenden Ansprüche,
**dadurch gekennzeichnet,**
**dass** der Beutelraum (12) mittels mindestens einer Verschlussvorrichtung (16) verschließbar ausgebildet ist.

4. Harnauffangbeutel nach Anspruch 3,
**dadurch gekennzeichnet,**
**dass** die Verschlussvorrichtung (16) aus mindestens einer in einer ersten Beutelwand ausgebildeten linienförmigen und hervorstehenden Prägung (28, 30) besteht, wobei die Prägung (28, 30) in eine entsprechende Nut in einer der ersten Beutelwand gegenüberliegenden zweiten Beutelwand lösbar eindrückbar ist.

5. Harnauffangbeutel nach einem der vorhergehenden Ansprüche,
**dadurch gekennzeichnet,**
**dass** der Harnauffangbeutel (10) mindestens einen Auslassanschluss (18) zum Anschluss eines Schlauchstücks (22) zur Ableitung des Harns aus dem Beutelraum (12) aufweist.

6. Harnauffangbeutel nach Anspruch 5,
**dadurch gekennzeichnet,**
**dass** der Auslassanschluss (18) oder das an dem Auslassanschluss (18) angeordnete Schlauchstück (22) mittels eines abnehmbaren oder nicht-abnehmbaren Clipverschluss (20) verschließbar ist.

7. Harnauffangbeutel nach Anspruch 5 oder 6,
**dadurch gekennzeichnet,**
**dass** das Schlauchstück (22) einstückig mit dem Auslassanschluss (18) verbunden ist.

8. Harnauffangbeutel nach einem der vorhergehenden Ansprüche,
**dadurch gekennzeichnet,**
**dass** der Einlassanschluss (14) ein Ventil zur Verhinderung eines Harnrückflusses aus dem Beutelraum (12) aufweist.

9. Harnauffangbeutel nach einem der vorhergehenden Ansprüche,
**dadurch gekennzeichnet,**
**dass** der Beutelraum (12) bodenseitig gerundete Eckbereiche (32, 34) aufweist.

10. Harnauffangbeutel nach einem der vorhergehenden Ansprüche,
**dadurch gekennzeichnet,**
**dass** der Harnauffangbeutel (10) in einem den Beutelraum (12) nach oben abschließenden Bereich mindestens eine Öffnung (24) zur Aufnahme einer hakenförmigen Halterung aufweist.

11. Harnauffangbeutel nach einem der vorhergehenden Ansprüche,
**dadurch gekennzeichnet,**
**dass** der Harnauffangbeutel (10) in einem den Beutelraum (12) nach oben abschließenden Bereich mindestens eine überstehende Bedienlasche (26) aufweist.

## Claims

1. Urine receptacle with at least one bag space (12) for receiving the urine and at least one inlet port (14) opening into the bag space (12), wherein the bag space is seamlessly formed,
**characterized in that**
the bag space (12) is formed reversible and is made of a flexible, liquid impermeable and film-like plastic material.

2. Urine receptacle according to claim 1,
**characterized in that**
the plastic material is a tubular film, wherein the tubular film is seamlessly welded on a bottom end of the bag space (12) for liquid-tight termination and for formation of the bag space (12).

3. Urine receptacle according to anyone of the preceding claims, **characterized in that**
the bag space (12) is formed closable by means of at least one closure device (16).

4. Urine receptacle according to claim 3,
**characterized in that**
the closure device (16) is constituted of at least one linear and protruding embossment (28, 30) formed in a first bag wall, wherein the embossment (28, 30) is capable of being detachably depressed into a corresponding groove in a second bag wall opposing the first bag wall.

5. Urine receptacle according to anyone of the preceding claims, **characterized in that**
the urine receptacle (10) has at least one outlet port (18) for connecting a piece of hose (22) for draining the urine from the bag space (12).

6. Urine receptacle according to claim 5,
**characterized in that**
the outlet port (18) or the piece of hose (22) disposed on the outlet port (18) is closable by means of a removable or non-removable clip fastener (20).

7. Urine receptacle according to claim 5 or 6,
**characterized in that**
the piece of hose (22) is integrally connected to the outlet port (18).

8. Urine receptacle according to anyone of the preceding claims, **characterized in that**
the inlet port (14) has a valve for preventing urine backflow from the bag space (12).

9. Urine receptacle according to anyone of the preceding claims, **characterized in that**
the bag space (12) has rounded corner regions (32, 34) at the bottom.

10. Urine receptacle according to anyone of the preceding claims, **characterized in that**
the urine receptacle (10) has at least one opening (24) for receiving a hook-shaped retainer in a region terminating the bag space (12) to the top.

11. Urine receptacle according to anyone of the preceding claims, **characterized in that**
the urine receptacle (10) has at least one protruding operating flap (26) in a region terminating the bag space (12) to the top.

## Revendications

1. Sac récupérateur d'urine avec au moins un espace formant sac (12), destiné à recueillir l'urine et au moins un raccord d'entrée (14) qui débouche dans l'espace formant sac (12), moyennant quoi l'espace formant sac est formé sans couture,
**caractérisé en ce**
**que** l'espace formant sac (12) est conçu de manière à pouvoir être retourné et se compose d'un matériau plastique souple, imperméable aux liquides et du type d'une feuille.

2. Sac récupérateur d'urine suivant la revendication 1,
**caractérisé en ce**
**que** le matériau plastique est une feuille extrudée en gaine, moyennant quoi la feuille extrudée en gaine est soudée sans joint, pour la fermeture étanche aux liquides et pour la formation de l'espace formant sac (12), sur une extrémité, côté fond, de l'espace formant sac (12).

3. Sac récupérateur d'urine suivant l'une des revendications précédentes,
**caractérisé en ce**
**que** l'espace formant sac (12) est formé de manière à pouvoir être fermé au moyen d'au moins un dispositif de fermeture (16).

4. Sac récupérateur d'urine suivant la revendication 3,
**caractérisé en ce**
**que** le dispositif de fermeture (16) se compose d'au moins une empreinte (28, 30), en forme de ligne et en saillie, formée dans une première paroi du sac, moyennant quoi l'empreinte (28, 30) peut être enfoncée de manière détachable dans une rainure correspondante dans une deuxième paroi du sac, opposée à la première paroi du sac.

5. Sac récupérateur d'urine suivant l'une des revendications précédentes,
**caractérisé en ce**
**que** le sac récupérateur d'urine (10) présente au moins un raccord de sortie (18), destiné au raccordement d'un élément tubulaire (22), destiné à évacuer l'urine hors de l'espace formant sac (12).

6. Sac récupérateur d'urine suivant la revendication 5,
**caractérisé en ce**
**que** le raccord de sortie (18) ou l'élément tubulaire (22), disposé sur le raccord de sortie (18) peut être fermé au moyen d'une fermeture clip (20) amovible ou non amovible.

7. Sac récupérateur d'urine suivant la revendication 5 ou 6, **caractérisé en ce**
**que** l'élément tubulaire (22) est relié d'un seul tenant au raccord de sortie (18).

8. Sac récupérateur d'urine suivant l'une des revendications précédentes,
**caractérisé en ce**
**que** le raccord d'entrée (14) présente une vanne, destinée à empêcher un reflux d'urine hors de l'espace formant sac (12).

9. Sac récupérateur d'urine suivant l'une des revendications précédentes,
**caractérisé en ce**
**que** l'espace formant sac (12) présente, côté fond, des zones angulaires arrondies (32, 34).

10. Sac récupérateur d'urine suivant l'une des revendications précédentes,
**caractérisé en ce**
**que** le sac récupérateur d'urine (10) présente, dans une zone terminant vers le haut l'espace formant sac (12), au moins une ouverture (24), destinée à loger une fixation en forme de crochet.

11. Sac récupérateur d'urine suivant l'une des revendications précédentes,
**caractérisé en ce**
**que** le sac récupérateur d'urine (10) présente, dans une zone terminant vers le haut l'espace formant sac (12), au moins une attache de manoeuvre (26) en saillie.
